# EUROPÄISCHE PATENTANMELDUNG

(11) **EP 1 435 224 A1**
(43) Veröffentlichungstag der Anmeldung: **07.07.2004**
(21) Anmeldenummer: 03450284.9
(22) Anmeldetag: 23.12.2003
(51) Int. Cl.: A61G 10/00, A61H 33/06, A61F 7/00

(54) **Einrichtung zur Behandlung eines Menschen**

(30) Priorität: 02.01.2003 AT 62003
(71) Anmelder: Leitner, Florian, 8770 St. Michael (AT)
(72) Erfinder: Leitner, Florian, 8770 St. Michael (AT)
(74) Vertreter: Gibler, Ferdinand, Dipl.Ing. Dr.

(57) **Zusammenfassung**

Transportable, auf eine Liegefläche (8) aufsetzbare Einrichtung zur Behandlung eines Menschen mit einer vorbestimmten Atmosphäre, welche Einrichtung einen Mantel (1) aufweist, der an seinen beiden Enden im wesentlichen abschließbar ist, wobei an einem Ende eine im wesentlichen dem Halsbereich eines Menschen entsprechende Ausnehmung (7) und ein Anschluss (10) zum Einbringen der vorbestimmten Atmosphäre vorgesehen ist. Um eine einfache Handhabung zu ermöglichen, ist vorgesehen, dass der Mantel (1) aus einem festen durchscheinenden Kunststoff, z.B. Acryl hergestellt ist und an seinen beiden Enden mit einem nach außen vorspringenden Flansch (2) versehen ist, an dem ein stimseitiger Abschluss (5, 6; 5') befestigbar ist.

## Beschreibung

Die Erfindung betrifft eine transportable, auf einer Liegefläche aufsetzbare Einrichtung gemäß dem Oberbegriff des Anspruches 1.

Derartige Einrichtungen werden zur meist partiellen Behandlung von Menschen mit einer vorbestimmten Atmosphäre, z.B. Warmluft, Dampf-Luftgemisch, Kaltluft oder auch mit bestimmten ätherischen Ölen oder Essenzen versetzte Luft, verwendet, wobei durch die Einrichtung ein Behandlungsraum mit relativ kleinem Volumen festgelegt wird und der Kopf des Patienten meist außerhalb dieses Behandlungsraumes verbleibt.

Bekannte derartige Einrichtungen weisen einen zeltartigen Aufbau auf, wobei eine entsprechend zugeschnittene Gewebebahn über ein auf der Liegefläche aufgestelltes Stützgerüst gespannt wird.

Dabei ergibt sich jedoch der Nachteil, dass die Aufstellung eines solchen aus Streben aufgebauten Stützgerüsts einen erheblichen Zeitaufwand bedingt und einiges Geschick erfordert. Außerdem muss die Gewebebahn dicht ausgebildet sein, um einen zu hohen Verlust der vorbestimmten Atmosphäre zu vermeiden. Aus diesem Grund werden solche Gewebebahnen in der Regel gummiert, wodurch diese aber undurchsichtig sind. Insbesondere bei Behandlungen mit Dampf stellt die hohe Flexibilität der Gewebebahn ein erhebliches Problem hinsichtlich der Anbringung der entsprechenden Anschlüsse dar. Außerdem stellt auch die Einhaltung der hygienischen Vorschriften bei der Reinigung des Stützgerüstes und der Gewebebahn ein erhebliches Problem dar, insbesondere, da die Streben des Stützgerüstes meist in nur schwer reinigbare kleine Taschen der Gewebebahn eingesteckt werden.

Ziel der Erfindung ist es, diese Nachteile zu vermeiden und eine Einrichtung der eingangs erwähnten Art vorzuschlagen, bei der diese Nachteile vermieden sind und die sich durch einen einfachen Aufbau auszeichnet.

Erfindungsgemäß wird dies bei einer Einrichtung der eingangs erwähnten Art durch die kennzeichnenden Merkmale des Anspruches 1 erreicht.

Durch die vorgeschlagenen Maßnahmen erübrigt sich das Aufstellen eines Stützgerüstes und es ist auch eine sehr einfache Reinigung unter Bedachtnahme auf die hygienischen Vorschriften möglich. So werden durch die vorgesehenen Flansche, an denen eine stirnseitige Abschlusswand befestigbar ist, schwer reinigbare Kanten vermieden. Dabei kann bei der Reinigung die Stirnwand abgenommen und separat gereinigt werden. Außerdem ist durch die Verwendung eines durchscheinenden, vorzugsweise durchsichtigen, Kunststoffs eine Beobachtung des Patienten möglich.

Im Hinblick auf eine leichte und effektive Reinigung ist es vorteilhaft die Merkmale des Anspruches 2 vorzusehen, da auf diese Weise innere Kanten, die bei der Reinigung entsprechende Problemstellen darstellen, vermieden sind. Dabei kann durch die Anordnung von Versteifungsrippen der Materialeinsatz und damit das Gewicht der Einrichtung entsprechend reduziert werden, wodurch die Einrichtung leicht transportabel ist.

Durch die Merkmale des Anspruches 3 kann die Einrichtung für den jeweiligen Einsatz entsprechend zusammengestellt werden. Dabei ergibt sich nicht nur der Vorteil eines entsprechend geringen Gewichtes der einzelnen Teile der Einrichtung und damit eine leichtere Handhabbarkeit, sondern auch der Vorteil, dass bei vielen Anwendungen der Behandlungsraum sehr klein gehalten werden kann, wodurch der Einsatz an vorbestimmter Atmosphäre ebenfalls gering gehalten werden kann.

Durch die Merkmale des Anspruches 4 kann die Einrichtung einfach und bequem auf die für einen zu behandelnden Menschen nötige Länge ausgezogen werden und nach Gebrauch auf einen kleinen Raum zusammengeschoben werden, sodass eine derartige Einrichtung zwischen platzsparend untergebracht werden kann. Dichtungen, wie z.B. lippenförmige Dichtungen, gewährleisten eine ausreichende Dichte zur Aufrechterhaltung einer gewünschten Atmosphäre während die Verschiebbarkeit der beiden Teile nicht beeinträchtigt wird.

Durch die Merkmale des Anspruches 5 ergibt sich eine einfache Verbindung der beiden Teile des Mantels, wobei durch die Vermeidung von Bohrungen, die schwer reinigbar sind, auch hohen Ansprüchen an die Hygiene entsprochen werden kann.

Durch die Merkmale des Anspruches 6 und 7 lässt sich auf einfache Weise ein rasch montierbarer und entfernbarer stirnseitiger Abschluss des Mantels und damit des Behandlungsraumes erreichen. Dabei ist auch im Falle eines Abschlusses mit einer Gewebebahn aufgrund der vorgeschlagenen Befestigung eine einfache und auch hohen Ansprüchen gerechtwerdende Reinigung möglich, da eben der Zuschnitt keine eingenähten Taschen oder dergleichen benötigt, die bei der Reinigung stets Problemzonen darstellen.

Durch die Merkmale des Anspruches 8 ist eine sichere Einleitung der vorbestimmten Atmosphäre auch dann möglich, wenn dabei auch Dampf eingebracht wird. Dabei ist es auch möglich gleichzeitig Dampf und Luft, insbesondere Warmluft in den Behandlungsraum einzubringen, wodurch eine stärkere Kondensatbildung an der Innenseite des Mantels und damit ein Abtropfen des Kondensats vermieden wird.

Durch die Merkmale des Anspruches 9 wird eine effiziente Beheizung bzw. Trocknung der Atmosphäre ermöglicht.

Durch die Merkmale des Anspruches 10 kann eine besonders homogene Atmosphäre für den Behandlungsraum aufbereitet werden.

Durch die Merkmale des Anspruches 11 kann eine Atmosphäre mit gewünschter Temperatur und/oder Luftfeuchtigkeit besonders genau und zuverlässig eingestellt werden.

Durch die Merkmale des Anspruches 12 lassen sich besonders wirkungsvolle Behandlungsprozeduren mit über die Zeit wechselnden Atmosphären durchführen.

Durch die Merkmale des Anspruches 13 wird auf einfache Weise eine gute Abdichtung nach außen erreicht und damit der Aufwand für die vorbestimmte Atmosphäre reduziert.

Durch die Merkmale des Anspruches 14 lässt sich zwischen Behandlungen auch die Liegefläche besonders platzsparend unterbringen.

Durch die Merkmale des Anspruches 15 ist der Ein- und Ausstieg in den Behandlungsraum besonders bequem durchführbar.

Die Erfindung wird unter Bezugnahme auf die beigeschlossenen Zeichnungen, in welchen Ausführungsformen dargestellt sind, näher beschrieben. Dabei zeigt:
Fig. 1 schematisch eine perspektivische Ansicht einer erfindungsgemäßen Einrichtung,
Fig. 2 und 3 eine vordere und eine hintere Stirnseite der Einrichtung nach der Fig. 1,
Fig. 4 einen vorderen Abschluss einer weiteren Ausführungsform einer erfindungsgemäßen Einrichtung,
Fig. 5 und 6 weitere Ausführungsformen einer erfindungsgemäßen Einrichtung,
Fig. 7 eine Verbindung zweier Mantelteile einer erfindungsgemäßen Einrichtung in größerem Maßstab,
Fig. 8 einen Anschluss zum Einbringen von Dampf und Luft in größerem Detail, und
Fig. 9 eine weitere Ausführungsform einer erfindungsgemäßen Einrichtung.
Fig. 10 eine weitere Ausführungsform einer erfindungsgemäßen Einrichtung.

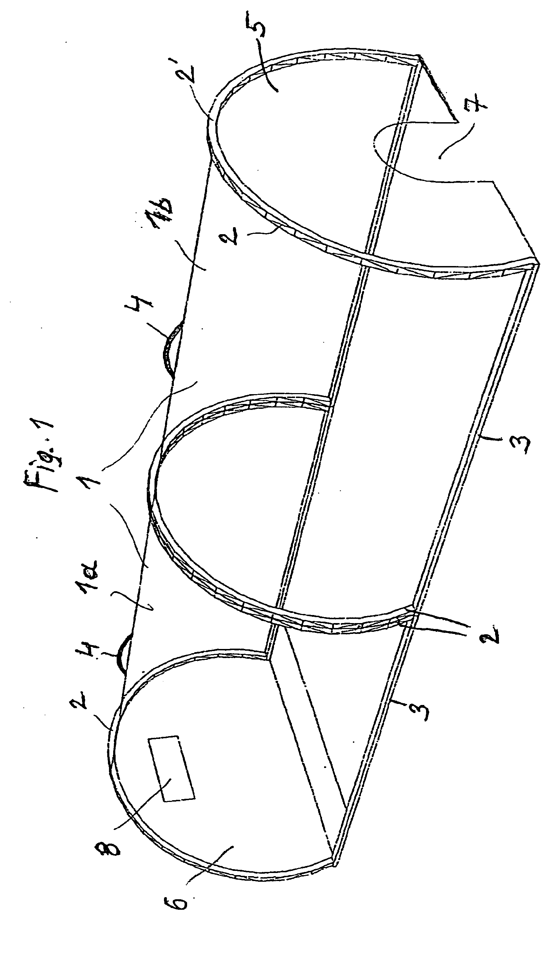

Wie aus der Fig. 1 zu ersehen ist, weist eine erfindungsgemäße Einrichtung im wesentlichen einen Mantel 1 auf, der beim dargestellten Ausführungsbeispiel zweiteilig ausgebildet ist. Dabei weist jeder der beiden Teile 1a, 1b an seinen beiden Enden einen nach außen gerichteten Flansch 2 auf, wobei die Mantelteile aus einem durchsichtigen Kunststoff hergestellt sind und eine Umfangslinie aufweisen, die im wesentlichen einem Kreisbogen entspricht, der sich über einen Winkel von mehr als 180° erstreckt.

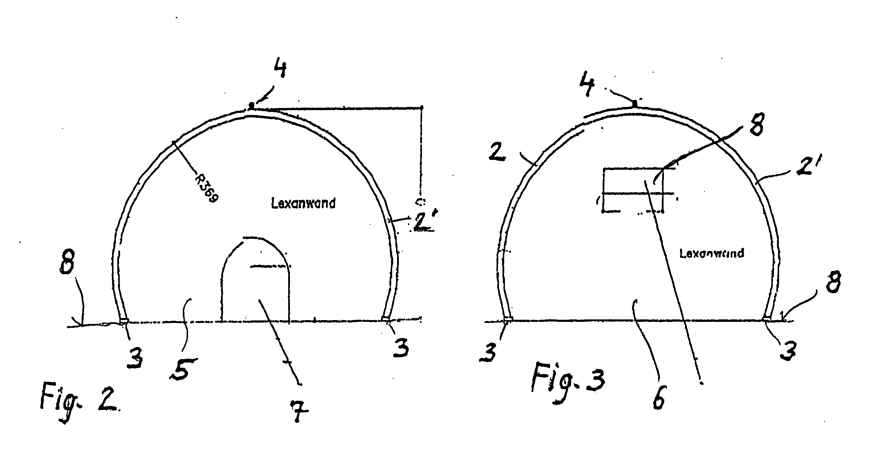

An den Längsrändern, die sich in Richtung der Mantellinien der beiden Mantelteile 1a, 1b erstrecken, sind Gummischienen 3 angebracht, mit denen die Mantelteile 1a, 1b auf einer in der Fig. 1 nicht dargestellten Liegefläche 8 (Fig. 2, 3) für einen Patienten aufliegen.

Diese Mantelteile 1a, 1b können mit Handgriffen 4 versehen sein, die einen leichten Transport der Mantelteile 1a, 1b ermöglichen.

An den beiden stirnseitigen Enden der Einrichtung sind Abschlüsse vorgesehen, die beim in der Fig. 1 dargestellten Ausführungsbeispiel durch Platten 5, 6 gebildet sind, die lösbar mit den Mantelteilen 1a, 1b verbunden sind. Dabei weisen die Platten 5, 6 eine Umfangslinie auf, die im wesentlichen der äußeren Umfangslinie der Flansche 2 und einer Sehne folgt, die die Längsränder der Mantellteile 1a, 1b verbindet. Die Platte 5 weist eine randoffene Ausnehmung 7 auf, die im wesentlichen dem Halsbereich eines Patienten frei lässt und die Platte 6 ist mit einem abdeckbaren Ausschnitt 8 versehen, der einen Regulierung der Durchströmung des Inneren der Einrichtung, das einen Behandlungsraum bestimmt, mit einer vorbestimmten Atmosphäre ermöglicht, die über eine einen der Fig. 1 nicht dargestellten Anschluss, in den Behandlungsraum einbringbar ist.

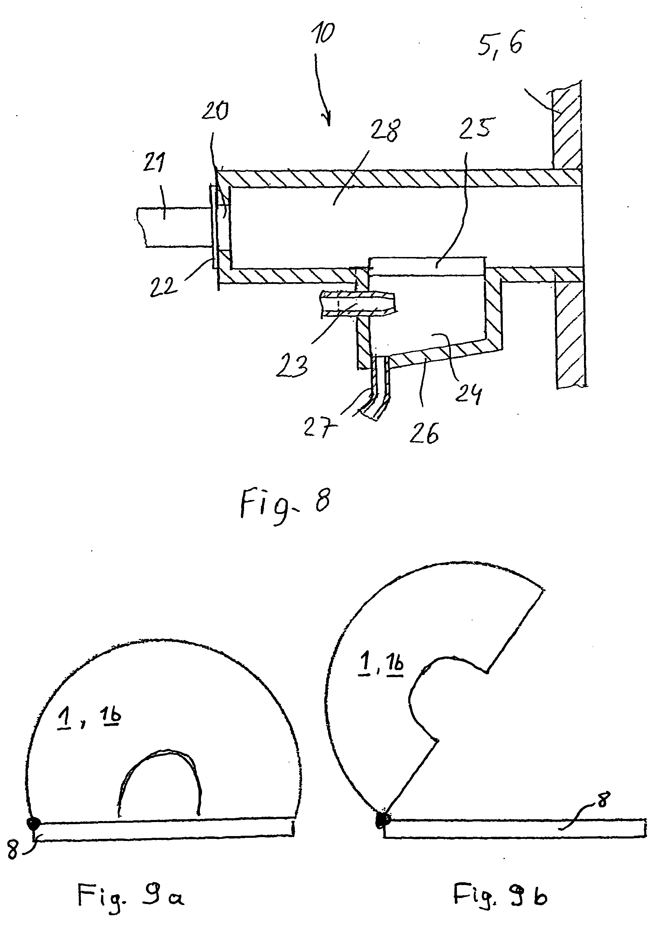

Die Platten 5, 6 sind mit einer den Flanschen 2 der Mantelteile 1a, 1b entsprechenden Verstärkung 2' versehen und mit den Flanschen verbindbar, wobei eine vorteilhafte Möglichkeit einer solchen Verbindung in der Fig. 7 schematisch dargestellt ist.

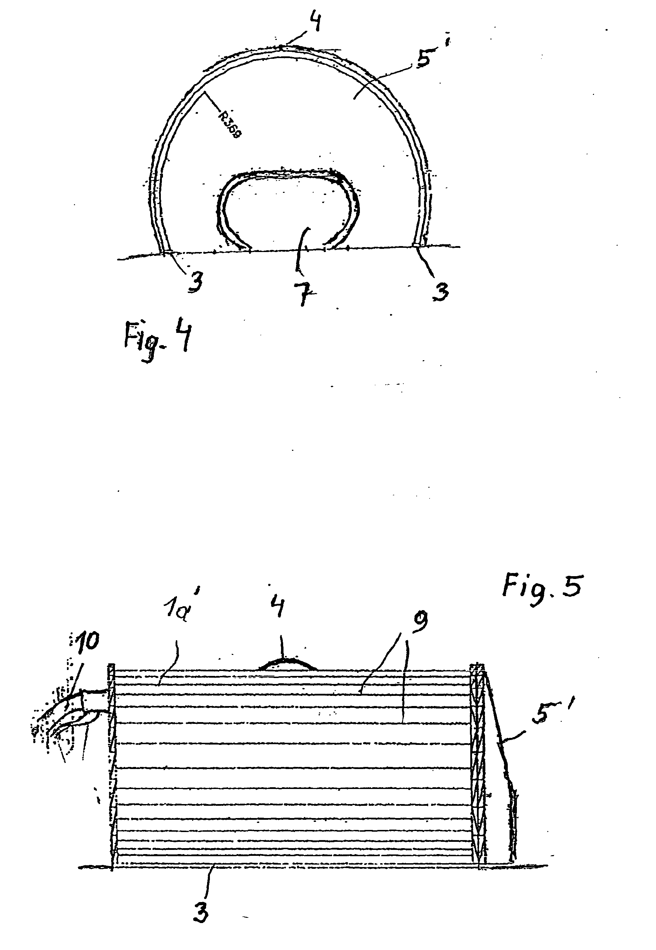

Eine weitere Möglichkeit eines stirnseitigen Abschlusses ist in den Fig. 4 und 5 dargestellt. Bei diesem Abschluss ist ein Zuschnitt 5' aus einem dichten, vorzugsweise gummierten Gewebe vorgesehen, der über den Flansch 2 des entsprechenden Mantelteiles 1a, 1b gefaltet ist und mit einem nicht dargestellten Gummizug befestigt ist. Weiters weist der Zuschnitt 5' einen Ausschnitt 7 auf.

Die Fig. 5 zeigt eine weitere Ausführungsform, bei der der Mantelteil 1a' mit in dessen Längsrichtung verlaufenden Versteifungsrippen 9 versehen ist, wobei diese Versteifungsrippen gerundet sind, etwa in der Art eines Wellbleches, um schwer reinigbare Kanten zu vermeiden. Der Mantelteil 1a' kann auch ein glatter Glas- oder Plexyglaszylinder sein, der sich durch bessere Transparenz und noch leichtere Reinigung auszeichnet. Weiters ist in der Fig. 5 ein Anschluss 10 zum Einbringen einer vorbestimmten Atmosphäre in den durch das Innere der Einrichtung bestimmten Behandlungsraum schematisch dargestellt. Dieser Anschluss 10 ist in der Fig. 8 näher dargestellt.

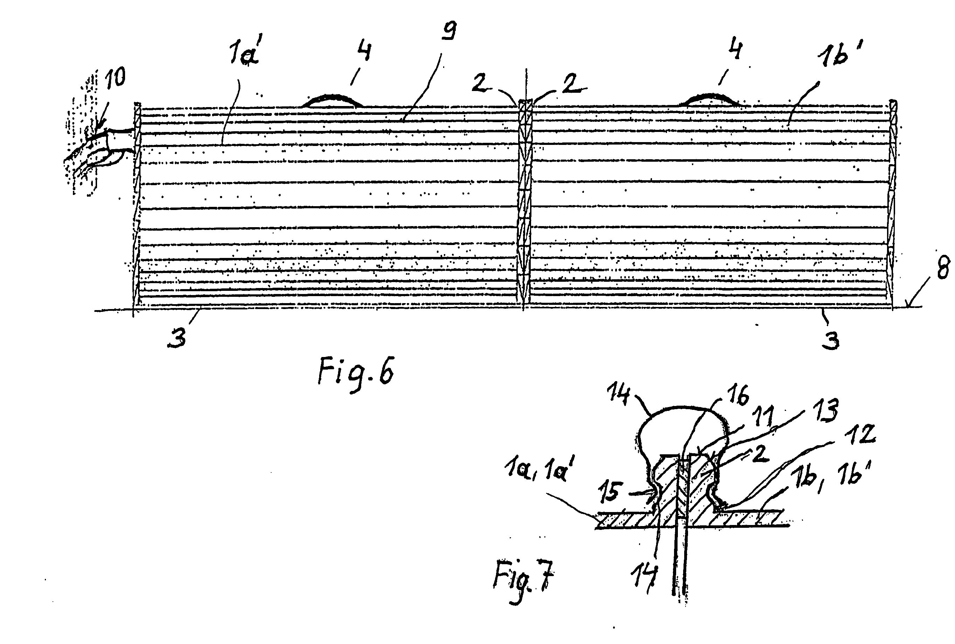

In der Fig. 6 ist ein Beispiel einer erfindungsgemäßen Einrichtung dargestellt, bei der zwei mit Versteifungsrippen 9 versehene Mantelteile 1a' und 1b' miteinander verbunden sind.

Die Verbindung ist in größerem Detail in der Fig. 7 gezeigt.

Die Flansche 2 der Mantelteile 1a, 1b; 1a', 1b' weisen eine an ihrer äußeren Mantelfläche 11 beginnende und zu deren hinterer Ringfläche 12 führenden Abschrägung 13 auf, die ein Aufsetzen einer federnden Klammer 14 erleichtert. Weiters ist in die hintere Ringfläche 12 eines jeden Flansches 2 und einer jeden Verstärkung 2' einer Platte 5, 6 eine Rille 14 eingearbeitet, in die eine im wesentlichen gegengleiche Rastnase 15 der im Querschnitt im wesentlichen lyraförmigen federnden Klammer 14 einrastet, wodurch ein sicherer Halt gewährleistet ist. Zwischen die Flansche 2 der beiden Mantelteile 1a', 1b' ist eine Flachdichtung 16 zwischengelegt, wodurch Rillen vermieden werden, die bei der Reinigung problematisch sind. Durch geeignet geformte unterschiedliche Ränder der Mantelteile 1a' und 1b' kann auch erreicht werden, dass diese ineinander gesteckt werden, sodass eine zusätzliche Dichtung vermieden werden kann.

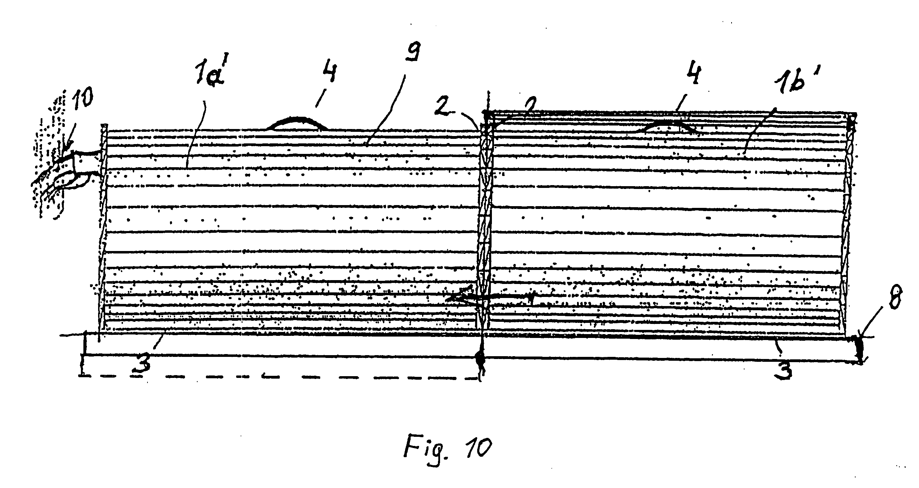

In Fig. 9a und 9b ist ein Beispiel einer erfindungsgemäßen Einrichtung dargestellt, bei der der Mantel 1 bzw. Mantelteile 1a, 1b; 1a', 1b' gelenkig an der Liegefläche 8 gelagert ist oder sind. Durch eine eingebaute Federung, vorzugsweise mit Stoßdämpfung, kann der Mantel 1 in geöffneter Position (siehe Fig. 9b) verbleiben, sodass ein bequemer Ein- bzw. Ausstieg möglich wird.

In der Fig. 10 ist ein Beispiel einer erfindungsgemäßen Einrichtung dargestellt, bei der zwei Mantelteile 1a' und 1b' ineinander verschiebbar angeordnet sind. In der dargestellten Ausführungsform weist der eine Mantelteil 1a' einen etwas geringeren Durchmesser auf als der andere Mantelteil 1b', sodass letzterer über ersteren drübergeschoben werden kann, wie durch den Pfeil angedeutet. Weiters ist in Fig. 9 strichliiert angedeutet, dass die Liegefläche 8 zweigeteilt bzw. zusammenklappbar ausgeführt sein kann.

Der Anschluss 10 weist, wie aus der Fig. 8 zu ersehen ist, einen Luftanschluss 20 auf, an den z.B. ein Schlauch 21 mittels eines Flansches 22 anschließbar ist, und in eine Mischkammer 28 mündet. Über diesen Anschluss 20 kann je nach Bedarf, Heiß-, Warm- oder Kaltluft eingebracht werden, oder auch mit bestimmten ätherischen Ölen oder Essenzen angereicherte Luft. Weiters weist der Anschluss 10 einen Dampfanschluss 23 auf, der in eine unterhalb einer Mischkammer 28 angeordnete Kammer 24 mündet, die über eine Öffnung 25 mit der Mischkammer 28 verbunden ist.

Diese Anordnung bedingt eine entsprechende Ablenkung des einströmenden Dampfes, wodurch Kondensat ausfällt und über den schrägen Boden 26 der Kammer 24, die als Kondensatrücklauf dient, zu einem Kondensatablauf 27 fließt.

Zweckmäßigerweise wird in den Behandlungsraum ein Dampf-Warmluftgemisch eingebracht, wobei der Dampf von der Heiß- bzw. Warmluft mitgerissen wird.

Zur präzisen Einstellung von Temperatur und Luftfeuchtigkeit der Atmosphäre kann eine Steuerung für die Zufuhr von Luft bzw. von Dampf vorgesehen sein. Eine solche Steuerung kann insbesondere über einen Computer erfolgen. Weiters kann diese Steuerung mit einem Ablaufprogramm gekoppelt sein, sodass Behandlungsprogramme mit über die Zeit wechselnden Atmosphären gefahren werden können.

## Patentansprüche

1. Transportable, auf eine Liegefläche (8) aufsetzbare Einrichtung zur Behandlung eines Menschen mit einer vorbestimmten Atmosphäre, welche Einrichtung einen Mantel (1) aufweist, der an seinen beiden Enden im wesentlichen abschließbar ist, wobei an einem Ende eine im wesentlichen dem Halsbereich eines Menschen entsprechende Ausnehmung (7) und ein Anschluss (10) zum Einbringen der vorbestimmten Atmosphäre vorgesehen ist, **dadurch gekennzeichnet, dass** der Mantel (1) aus einem festen durchscheinenden Kunststoff, z.B. Acryl hergestellt ist und an seinen beiden Enden mit einem nach außen vorspringenden Flansch (2) versehen ist, an dem ein stirnseitiger Abschluss (5, 6; 5') befestigbar ist.

2. Einrichtung gemäß Anspruch 1, **dadurch gekennzeichnet, dass** die Umfangslinie des Mantels (1) im wesentlichen einem Kreisbogen entspricht, wobei der Mantel (1) vorzugsweise mit sich in dessen Längsrichtung erstreckenden Versteifungsrippen (9) mit gerundetem Querschnitt versehen ist.

3. Einrichtung gemäß Anspruch 1 oder 2, **dadurch gekennzeichnet, dass** die Einrichtung zweigeteilt ist, wobei jeder der beiden Teile des Mantels (1a, 1b; 1a', 1b') an seinen beiden Enden mit nach außen vorspringenden Flanschen (2) versehen ist, wobei die Flansche (2) der beiden Teile (1a, 1b; 1a', 1b') des Mantels (1) die gegebenenfalls unter Zwischenlage einer Dichtung (16), vorzugsweise einer Flachdichtung, aneinander zur Anlage bringbar und miteinander verbindbar sind.

4. Einrichtung gemäß Anspruch 1 oder 2, **dadurch gekennzeichnet, dass** die Einrichtung zweigeteilt ist, wobei die beiden Teile des Mantels (1a, 1b; 1a', 1b') ineinander verschiebbar angeordnet sind, wobei gegebenenfalls eine Dichtung (16), vorzugsweise eine lippenförmige Dichtung, zwischen den beiden Teile (1a, 1b; 1a', 1b') des Mantels (1) angeordnet ist.

5. Einrichtung gemäß einem der Ansprüche 1 bis 3, **dadurch gekennzeichnet, dass** die Flansche (2) an ihrer von ihrer freien Stirnseite abgekehrten hinteren Ringfläche (12) eine an deren Mantelfläche (11) beginnende Abschrägung (13) aufweisen und die beiden Mantelteile (1a, 1b; 1a', 1b') mittels Federklammern (16) verbindbar sind, die vorzugsweise in eine an der hinteren Ringfläche (12) der Flansche (2) eingearbeitete, im wesentlichen parallel zur Umfangslinie des Mantels (1) verlaufende Nut (14) einrasten.

6. Einrichtung gemäß einem der Ansprüche 1 bis 5, **dadurch gekennzeichnet, dass** der stirnseitige Abschluss durch eine Platte (5, 6) gebildet ist, deren Umfangslinie im wesentlichen jener der äußeren Umfangslinie der Flansche (2) und einer Sehne entspricht, die die Längsränder der Mantelteile (1a, 1b; 1a', 1b') verbindet.

7. Einrichtung gemäß einem der Ansprüche 1 bis 6, **dadurch gekennzeichnet, dass** der stirnseitige Abschluss durch einen Zuschnitt (5') aus einem gummierten Gewebe hergestellt ist, der vorzugsweise mit einem einen Flansch (2) des Mantels (1) übergreifenden Gummizug befestigbar ist.

8. Einrichtung gemäß einem der Ansprüche 1 bis 7, **dadurch gekennzeichnet, dass** der Anschluss (10) zur Einbringung der vorbestimmten Atmosphäre einen Luftanschluss (20) und einen Dampfanschluss (23) aufweist, dem ein Kondensatfänger unmittelbar nachgeordnet ist.

9. Einrichtung gemäß Anspruch 8, **dadurch gekennzeichnet, dass** der Luftanschluss (20) für Heißluft, insbesondere im Bereich zwischen 60 und 90°C ausgelegt ist.

10. Einrichtung gemäß Anspruch 8 oder 9, **dadurch gekennzeichnet, dass** eine Mischkammer (28) vorgesehen ist, in die der Luftanschluss (20) und der Dampfanschluss (23) einmünden.

11. Einrichtung gemäß einem der Ansprüche 8 bis 10, **dadurch gekennzeichnet, dass** die Zufuhr von Luft, insbesondere Heißluft, über den Luftanschluss (20) und/oder die Zufuhr von Dampf über den Dampfanschluss (23) insbesondere über einen Computer steuerbar ist.

12. Einrichtung gemäß Anspruch 11, **dadurch gekennzeichnet, dass** die Zufuhr von Luft, insbesondere Heißluft, über den Luftanschluss (20) und/oder die Zufuhr von Dampf über den Dampfanschluss (23) nach einem vorgebbaren Temperatur-Feuchtigkeits-Programm steuerbar ist.

13. Einrichtung gemäß einem der Ansprüche 1 bis 12, **dadurch gekennzeichnet, dass** an den beiden Längskanten des Mantels (1) Gummischienen (3) angebracht sind.

14. Einrichtung gemäß einem der Ansprüche 1 bis 13, **dadurch gekennzeichnet, dass** die Liegefläche (8) in wenigstens zwei Teile teilbar bzw. zusammenklappbar ausgeführt ist.

15. Einrichtung gemäß einem der Ansprüche 1 bis 14, **dadurch gekennzeichnet, dass** der Mantel (1) bzw. die Mantelteile (1a, 1b; 1a', 1b') an jeweils einer Längskante gelenkig, vorzugsweise gefedert und/oder stoßgedämpft, mit der Liegefläche (8) verbunden ist bzw. sind.
